(19)

Europäisches
Patentamt
European
Patent Office
Office européen
des brevets

(11)     **EP 2 186 542 A1**

(12)     **EUROPEAN PATENT APPLICATION**

(43) Date of publication:
**19.05.2010 Bulletin 2010/20**

(51) Int Cl.:
***A61N 5/10*** (2006.01)

(21) Application number: **08169193.3**

(22) Date of filing: **14.11.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **ION BEAM APPLICATIONS S.A.**
**1348 Louvain-la-Neuve (BE)**

(72) Inventors:
• **Labarbe, Rudi**
  **B-4100, BONCELLES (BE)**
• **Bentefour, El Hassane**
  **B-3300, TIENEN (BE)**

(74) Representative: **pronovem**
**Office Van Malderen**
**Avenue Josse Goffin 158**
**1082 Bruxelles (BE)**

(54)     **Method and device for determining a radiation isocenter in a particle therapy room**

(57)     The present invention relates to a method and device for determining the position of a radiation isocenter (101,102) of a particle beam delivery system (500). The particle beam delivery system (500) is configured for delivering a radiation beam (2001, 2002) along one or a multiple number of delivery directions ($D_1$, $D_2$, ...., $D_i$, ..., $D_n$) in a treatment room. The position of the radi-
ation isocenter (101,102) is being determined for each delivery direction. The device of invention comprises two beam monitors (10,20) arranged in a face to face relationship at a given distance from each other. Both beam monitors (10,20) are measuring a radiation field in two dimensions. Processing means are provided to calculate the position of the radiation isocenter (101,102) with respect to a reference 3D coordinate system (150).

Fig. 1

EP 2 186 542 A1

**Description**

**TECHNICAL FIELD**

The invention relates to the field of charged particle radiation therapy. More particularly, the present invention relates to a method and device for determining the position of a radiation isocenter with respect to a reference coordinate system.

**BACKGROUND OF THE INVENTION AND DEFINITIONS USED IN THE PRESENT TECHNOLOGICAL FIELD**

Radiotherapy using charged particles has proven to be a precise and conformal radiation therapy technique where a high dose to a target volume can be delivered while minimizing the dose to surrounding healthy tissues.

Typically, a particle therapy apparatus comprises an accelerator producing a beam of energetic charged particles, a beam transport system to guide the particle beam to a therapy room, a particle beam delivery system and means to position a patient using for example a therapy couch.

The particle beam delivery system is able to deliver radiation beams and thereby is responsible to provide a conformal dose distribution to a target volume. Said particle beam delivery system is also able to monitor and measure the dose delivered.

A radiation beam can be defined as a spatial region containing a flux of charged particles.

Beam delivery systems generally generate divergent radiation beams where the charged particles originate from a virtual radiation source.

The virtual radiation source is defined as the point from which the radiation appears to originate from. A divergent radiation beam can for example have the shape of an infinite cone, i.e. a cone that has no bounding base and extends to infinity. Other type of beam delivery systems for particle therapy can deliver parallel radiation beams where the virtual source is at infinite distance. In such a case the radiation beam can for example take the shape of a cuboid. For patient treatment however, the radiation beams will in general not have a geometrical shape but the beam delivery system will create an appropriate shape adapted to the form of the specific target volume to be irradiated.

Hence, the term 'radiation beam axis' is intended to mean the central beam trajectory line followed by the particles. For example for a radiation beam having a right circular conical shape in an orthogonal plane to said beam, the radiation beam axis is passing through the virtual source (apex of the cone) and corresponds to the axis of rotational symmetry of the cone.

When intersecting a radiation beam with a surface, the area on the surface where the radiation intensity exceeds a specific or specified level is generally called a radiation field.

A radiation beam can for example be intersected with a beam monitor allowing to measure some characteristics of a radiation field such as for example the radiation field size, the uniformity of the dose across the radiation field, ....

The beam delivery system comprising a nozzle has either a fixed orientation in the treatment room to deliver the radiation beam to the target from a fixed direction or the beam delivery system can have multiple orientations in the treatment room.

The beam delivery system can for example be mounted on a gantry system which is a large structure capable of rotating the beam delivery system around e.g. a horizontal axis of rotation. By rotating the gantry, the beam delivery system can deliver radiation beams to the target volume from any direction in a vertical plane. In combination with the rotation of the therapy couch, it should be possible to irradiate the whole target volume when using a gantry system.

A variety of particle beam delivery systems exist, applying different techniques to deliver a conformal dose to the target volume. There are two major techniques used in particle beam delivery: the common passive scattering techniques and the more sophisticated dynamic radiation techniques.

An example of a dynamic technique is the so-called pencil beam scanning (PBS) technique. In PBS, the radiation beam is generated by magnetically scanning with an elementary pencil beam (generally having a Gaussian distribution with a sigma value typically between 3 to 10 mm) on the plane orthogonal to the radiation beam axis.

By properly defining the trajectory of the elementary pencil beam one can generate geometrical radiation beams resulting in geometrical field shapes (circles, squares, rectangulars, ...).

In the case of PBS, a radiation beam is defined as being the integration of all elementary pencil beams being scanned over a plane orthogonal to the radiation beam axis.

Similar one can say that the radiation field in PBS is the result from an integration of multiple elementary radiation fields delivered with an elementary pencil beam that is scanned over a surface.

For patient, treatment the radiation beams have in general not a geometric shape and the lateral dose conformity to the target volume, i.e. in a plane perpendicular to the radiation beam axis, is obtained by adequate control of the scanning magnets in order the elementary pencil beam to follow precisely the contour of the target volume.

By varying the energy of the particle beam, different layers in the target volume, characterised by their fixed particle energy, is subsequently irradiated. In this way, particle radiation dose can be delivered to the entire 3D target volume.

Another example of a dynamic particle radiation technique that differs from pencil beam scanning is the so called wobbling technique, also named uniform scanning technique, where a uniform dose is delivered to a target volume layer per layer and where each elementary beam continuously scan a geometrical scanning pattern.

As for PBS, in uniform scanning the integration of all delivered elementary beams builds up the radiation beam. The elementary beam in uniform scanning has generally a larger size than in PBS where a narrow pencil beam is used.

In uniform scanning, the elementary beam is scan a predefined geometrical area (square, rectangle, circle, ...) - different from geometrical section of the target volume - and lateral conformity, i.e. in a plane perpendicular to the radiation beam axis, is accomplished by using a multileaf collimator or a patient specific aperture.

The passive scattering beam technique is using scattering elements in the beam delivery system to laterally spread the beam. The use of one or two scatter elements corresponds to the so-called single-scattering and double-scattering beam delivery technique, respectively.

For spreading the beam along the beam direction the passive scattering technique is generally using a rotating range modulator. The radiation beams from a passive scattering system are divergent and the shape of the radiation beam is conical. To make the radiation beam conform to the target volume, a patient specific collimator and range compensator mounted on the beam delivery system can be used. The patient specific collimator adapts the lateral shape, ie in a plane perpendicular to the radiation beam axis, of the radiation beam to the contours of the target volume.

The beam delivery system delivers radiation beams that are specified by a treatment plan. In order not to damage healthy cells within the patient's body during the treatment, an accurate positioning of the patient is required, so that the beam only reaches the target volume as specified in the treatment planning.

The treatment plan generally uses an image of the patient comprising the target volume to be irradiated and the location of the treatment plan isocenter is defined on the images as part of the treatment planning process.

The treatment planning isocenter corresponds generally to the center of the target volume. Characteristics of the radiation field at the isocentric plane, i.e. the plan going through the isocenter and perpendicular to the radiation beam axis, are know by the treatment planning system. For example, the position of the virtual source is also known with respect to the isocentric plane.

At the day of treatment, the patient is first positioned and subsequently aligned - prior to irradiation - and the purpose of this alignment process is to align the patient such that the beam to target volume geometry is according to the treatment planning and the treatment plan isocenter coincides with the radiation isocenter of the beam delivery system.

Different techniques can be used to align the patient prior to irradiation. Typically X-ray images (DR's) are acquired and these images are compared with the images defined at the level of the treatment planning system (so-called, DRR's: Digitally Reconstructed Radiographs). The idea is then to match the DR's with the DRR's in order to have the target volume being positioned and oriented as specified by the treatment plan and have the treatment planning isocenter coinciding with the radiation isocenter of the beam delivery system.

For effective usage of particle therapy, a beam to target accuracy in the sub millimeter region is required.

In classical radiotherapy, the radiation isocenter of an isocentric gantry system is generally defined as the point in space where the radiation beam axes of radiation beams delivered from various gantry angles intersect. In practice, the radiation beam axes do not intersect in a single point but intersect in a small volume like a sphere or ellipsoid. Usually the center-of-mass of this volume is specified as the "common" radiation isocenter of the gantry system. There can be different reasons why the isocenter is not a single point for all gantry angles.

For example, the gantry geometry changes as function of the gantry angle (gantry sag) resulting in a different radiation isocenter for each gantry angle.

The alignment of the different elements in the beam delivery system can influence the position of the radiation isocenter.
For example, the radiation isocenter is influenced by the alignment of the scanning magnets in a PBS nozzle or by the position of the collimator and other elements in a passive scattering nozzle.

For effective usage of particle therapy, a beam to target accuracy in the sub millimeter region is required.

The use of a common radiation isocenter for all gantry angles is not suitable and therefore a radiation isocenter for each gantry angle needs to be determined.

Hereafter, the term "radiation isocenter" is intended to mean any radiation isocenter determined for a given gantry angle or more general for a beam delivery system delivering radiation beams from a given delivery direction.

The term 'common radiation isocenter' refers to one single radiation isocenter defined for a gantry system.

By defining the radiation isocenter of a beam delivery system for each potential beam direction, the term "radiation isocenter" can also be used in the context of a beam delivery system having only a single fixed direction.

For a particle therapy system providing multiple types of particles in the same treatment room (e.g. protons and carbon ions), the radiation isocenter can differ for each particle type and hence these radiation isocenters need to be determined.

In US 7349523, a method and device is disclosed to measure the common radiation isocenter of a radiation treatment device. The device makes use of a three-dimensional phantom and films or radiation sensitive sheets which or mounted around the phantom. The beam delivery system is configured to deliver a beam pattern resulting for each gantry angle in multiple lines to be observed on the film. A common radiation isocenter is determined based on an analysis of the position of the points of interception of the beam on two opposed films when the beam is coming from two different directions or trajectories. This device and method has several drawbacks and can not be used to determine the radiation isocenter of a particle therapy system with the accuracy required. The first problem is that film is used and for each gantry angle a multiple number of lines are generated on the film. For a few gantry angles (e.g. two or three) this technique can be applied as disclosed in the patent, however when for each gantry angle (from 1° to 360°) a radiation isocenter needs to be determined this method is not useful as it becomes difficult to find out on the films what lines correspond to what gantry angle. A second drawback is that the method is cumbersome due to the need to develop a multitude of films. Another problem is the fact that a specific collimator or device needs to be mounted on the beam delivery system to be able to create crossing lines on the film, i.e. the delivery machine is not operating in a standard treatment configuration when the radiation isocenter is being determined. Also, this method and device disclosed in this prior art patent aims at determining a common radiation isocenter being the optimum radiation isocenter for all treatment angles, i.e. it the device and method does not determine a radiation isocenter as function of gantry angle.

## SUMMARY OF THE INVENTION

The invention aims at providing a device and method to determine the beam or radiation isocenter of a particle therapy apparatus that do not present the drawbacks of the state of the art. In particular, the present invention aims at determining the coordinates of a beam isocenter in a treatment room for each available delivery direction. These coordinates are determined with respect to a reference coordinate system.

According to a first aspect of the present invention, a method is provided for determining the position of a radiation isocenter of a particle beam delivery system with respect to a 3D reference coordinate system, said particle beam delivery system being configured for delivering a radiation beam along one or a multiple number of delivery directions $(D_1, D_2, ...., D_i, ..., D_n)$ in a treatment room, the method comprising the steps of:

> a) positioning the particle beam delivery system in one of the delivery directions $(D_1, D_2, ...., D_i, ..., D_n)$;
> b) setting up at least two beam monitors downstream of the said particle beam delivery system,
> c) establishing the position and orientation of the said two beam monitors with respect to the 3D reference coordinate system;
> d) delivering said radiation beam;
> e) measuring detector responses of said two beam monitors;
> f) reconstructing the radiation beam axis of said radiation beam with respect to said 3D reference coordinate system;
> g) determining for each of said delivery directions $(D_1, D_2, ...., D_i, ..., D_n)$ the position of the corresponding said radiation isocenter with respect to said 3D reference coordinate system

According to first aspect of the invention the preferred embodiment further comprises steps of::

> a) defining the coordinates of the common radiation isocenter (M) for all beam delivery directions;
> b) computing the coordinates of the radiation isocenter for each said delivery directions by making an orthogonal projection of said common radiation isocenter (M) on the corresponding radiation beam axis.

Advantageously, according to the first aspect of the invention, the method further comprises steps of:

> a) defining the coordinates of the common radiation isocenter (M) for all beam delivery directions;
> b) determining the position of the said radiation isocenter by computing for each said beam delivery directions a correction vector with respect to said common radiation isocenter (M).

According to the first aspect of the invention, the method further comprises the step of:

> determining the position of said radiation isocenter for each said delivery directions as being the point on said radiation beam axis located at a given distance from said beam delivery system.

According to a second aspect of the present invention, a device is provided for determining the position of a radiation isocenter of a particle beam delivery system with respect to a 3D reference coordinate system, said particle beam delivery system being configured for delivering a radiation beam along one or a multiple number of delivery

directions ($D_1$, $D_2$, ...., $D_i$, ..., $D_n$) in a treatment room, the device being characterized in that it comprises:

o a beam monitor assembly comprising at least two beam monitors arranged in a face to face relationship at a given distance from each other, each of said two beam monitors being generating two-dimensional detector responses when said radiation beam is crossing the said two beam monitors;
o beam monitor electronics for performing data acquisition of said detector responses;
o means to set up the said beam monitor assembly downstream of the said particle beam delivery system in such a manner that said radiation beam is crossing the two said beam monitors when said particle beam delivery system is delivering said radiation beam along said delivery direction;
o processing means to reconstruct the radiation beam axis of said radiation beam with respect to said 3D reference coordinate system ;
o processing means for computing and determining for each of said delivery directions ($D_1$, $D_2$, ...., $D_i$, ..., $D_n$) the position of the corresponding said radiation isocenter with respect to said 3D reference coordinate system,, said computing using the said reconstructed radiation beam axis of each said delivery direction.

The beam monitor assembly can either be an integrated assembly comprising a frame to hold the two beam monitors together in a face to face relationship or the beam monitor assembly comprises two beam monitors which are mechanically not linked together. If the former case the beam monitor assembly can be supported by a single support frame (30) whereas in the latter case the two beam monitors can have each their proper independent support frame.

In the context of the present invention, the position of a radiation isocenter is entirely defined by its three coordinates X,Y,Z in a 3D reference coordinate system. If the beam delivery system can provide radiation beams from multiple directions (e.g. gantry system), the device of invention will determine for each delivery direction the absolute coordinates of the radiation isocenter in a 3D reference coordinate system. In practice, for a gantry system, the radiation isocenter is determined as function of the gantry angle. For an isocentric gantry system, instead of determining the absolute coordinates of the radiation isocenter for each gantry angle the device of invention allows also to determine in a first step the coordinates of a theoretical radiation isocenter (M) (common isocenter for each gantry angle) and in a second step the determination of the relative positions (correction vector) of the radiation isocenter for each gantry angle with respect to this common radiation isocenter.

Advantageously, the device wherein said processing means to reconstruct the radiation beam axis further comprises:

o processing means to analyse the said detector responses and to identify the intersection points of the radiation beam axis with said two beam monitors;
o processing means to compute and determine the position of the said intersection points with respect to the said 3D reference coordinate system.

Advantageously, each of said beam monitors comprises a two-dimensional detector.
More advantageously, said two-dimensional detector comprises a first strip electrode and a second strip electrode arranged in a face to face relationship and having respective strips oriented in two orthogonal directions whereby forming a matrix of orthogonal strips.
Preferably, the beam monitor assembly comprises reference markers for determining the position of the beam monitor assembly with respect to a 3D reference coordinate system. These reference markers can be targets for use with a theodolite.
Advantageously, said setup means comprises rotation means for adjusting the orientation of said beam monitor assembly with respect to the said delivery direction of said radiation beam.
Preferably, said rotation means comprises means for reading said angular position of the beam monitor assembly.
Optionally, said setup means is adapted to attach said beam monitor assembly directly to a patient couch.
Optionally, said setup means comprises adjusting means for adjusting the position of one of said monitors in a direction essentially perpendicular to the radiation beam axis of the said radiation beam.

## BRIEF DESCRIPTION OF THE DRAWINGS

**FIG. 1** is a perspective view of a device according to the invention.
**FIG. 2a** schematically represents the setup in a therapy room of the beam monitor assembly comprised in the device of invention. The beam delivery system (500) is oriented in the treatment room according to a direction $D_i$.
**FIG. 2b** schematically represents an alternative setup in a therapy room of the beam monitor assembly comprised in the device of invention. The beam delivery system (500) is oriented in the treatment room according to a

direction $D_j$.

**FIG. 3** is a front view in the direction of arrow A of Fig. 1 of the beam monitor assembly and electronics comprised in the device of the invention.

**FIG. 4** is an exploded view of a beam monitor of the device of **Fig. 1.**

**FIG. 5** is a front view of a strip electrode of the beam profile monitor of Fig.1.

**FIG. 6** is a perspective view of a second beam profile monitor of the device of **Fig.1.**

**FIG. 7a** shows an example of a radiation field as detected with a two dimensional beam monitor according to the invention.

**FIG. 7b** shows examples of lateral profiles of a radiation field as detected with a beam monitor according to the invention.

**FIG. 8** shows the beam profile monitor coordinate system (BPM Coordinate System) attached to the beam monitor assembly according to the invention..

**FIG. 9** represents an example of a graphical user interface for use in the invention.

**Fig. 10** shows three radiation beam axes ($T_i$) in the FRS (Fixed Reference System) and three corresponding radiation isocenters ($P_i$).

## DETAILED DESCRIPTION OF THE INVENTION

The present invention provides a device 1 and method to determine the coordinates of the radiation isocenter of a charged particle beam delivery system, delivering radiation beams to a target volume. The radiation isocenter is determined for all available beam delivery directions in the treatment room. For a beam delivery system based on a gantry, this means that the radiation isocenter is determined for all available gantry angles. The device is however not limited for use in a gantry treatment room but can also be used in other types of treatment rooms, for example a treatment room with one or more fixed beam lines.

The invention relates to the use of a method and device for calibrating a radiation therapy apparatus. Remark that the method and device of the present invention is used without any patient in the treatment room.

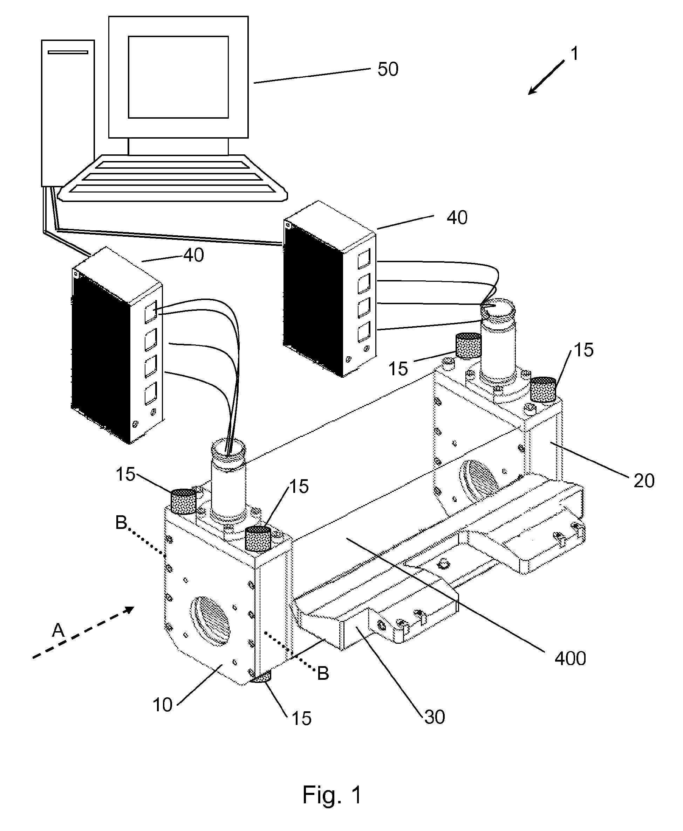

Fig. 1 represents a schematic view of a device according to a preferred embodiment of a first aspect of the present invention. There is a beam monitor assembly 400 comprising two beam monitors 10, 20 and means to hold the two beam monitors 10, 20 in a face to face relationship, at a defined distance from each other (for example 50 cm). The beam monitors 10, 20 are each linked to acquisition electronics 40 which are further connected to a computer 50 to process and analyse the recorded data. The support member 30 holds the detector assembly 400 in position during field irradiation measurements. As will be shown below, various embodiments for the support frame 30 can be developed. The support frame shown in Fig. 1, is a support member to be directly attached to a patient couch.

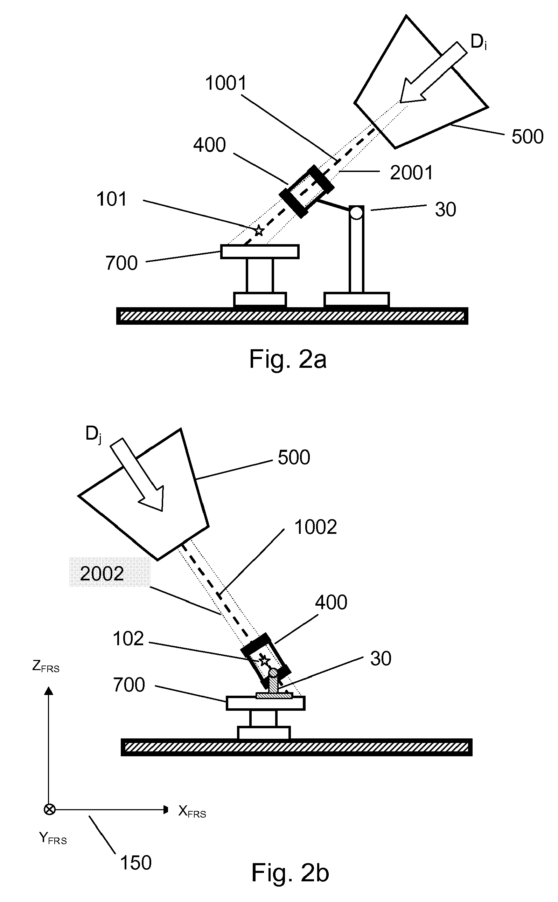

Figs. 2a and 2b are showing two examples of a setup arrangement for the two beam monitors 10, 20 in a treatment room. The two beam monitors 10, 20 are typically located between the patient couch 700 and the beam delivery system 500. The beam monitor assembly 400, supported by a support frame 30, is positioned within the therapy room in such a manner that the radiation beam 2001, 2002 can pass through both beam monitors 10, 20. In Fig. 2b the support member 30 is positioned on the patient couch whereas in Fig. 2a a separate support stand positioned on the floor is used as a support member 30. In Fig. 2a and Fig. 2b the delivery direction of the beam delivery system is $D_i$ and $D_j$, respectively. For a gantry system, these directions are defined by the respective gantry angles. The radiation beam 2001, 2002 delivered by the beam delivery system 500 is illustrated with pointed lines on Fig. 2. For both examples, the radiation beam axis 1001, 1002, defined as a line going through the center of the radiation beam 2001, 2002 is shown as a dotted line. The radiation isocenter 101, 102 is by definition a point located along the radiation beam axis. The radiation isocenter 101, 102 of the beam delivery system 500 as positioned at a given direction ($D_i$ and $D_j$) is schematically represented by a star.

The coordinates of the radiation isocenter 101,102 are determined according to a 3D reference coordinate system 150. This reference coordinate system can for example be the so-called Fixed Reference System (FRS) which is fix with respect to the treatment room.

As illustrated on Fig. 2b, for a gantry treatment room, , the Z-axis of the FRS is the vertical direction in the therapy room, the Y-axis is parallel with the rotation axis of the gantry and the X-axis is orthogonal to both Z and Y axis, in such a way to have a right handed coordinate system. The origin of the FRS is a reference point to be chosen in the treatment room.

However, the device 1 is not limited to the use of this FRS, any other reference coordinate system can be used as well, for example a coordinate system fixed to the patient couch or a coordinate system fixed to the gantry.

In a preferred embodiment of the present invention, the two beam monitors 10,20 can be oriented and aligned along the direction of the beam delivery system. For a gantry system rotating by 360°, this means that the two beam monitors will be mounted on a support frame 30 that allows to rotate the two monitors 10, 20 by 360° in order to bring the two monitors 10,20 in front of the beam delivery system for each gantry angle.

In a variant of this preferred embodiment the rotation mechanism allowing to rotate the monitor assembly may further comprise means for reading the angular orientation of the monitor assembly.

Furthermore, locking means are also provided for locking the beam monitor assembly at a fixed rotation angle.

In a second variant of this embodiment the beam monitor assembly comprises sliding means for allowing the beam monitor 20 translating laterally in the direction of line B of **Fig. 1** with a translation range of a several cm (for example ± 2 cm) and a resolution of the translation steps of at least 1 mm. This sliding means is provided for adjusting the relative position of beam profile monitor 20 with respect to the beam profile monitor 10 so that the radiation beam hits both beam monitors 10, 20. This feature for sliding the second beam monitor 20 can be used for example for a situation where the radiation beam axis is slightly tilted with respect to the plane of the monitor detectors.

In an alternative embodiment, both beam monitors 10, 20 can be equipped with lateral sliding means.

According to another variant of this preferred embodiment said sliding means comprises locking means for selectively blocking said beam monitor 10, 20 and preventing from laterally sliding.

Preferably the two beam monitors 10,20 are positioned parallel in a face to face relationship.

Alternatively, two monitors are not positioned parallel then the exact relative position and geometry between the two beam monitors needs to be determined.

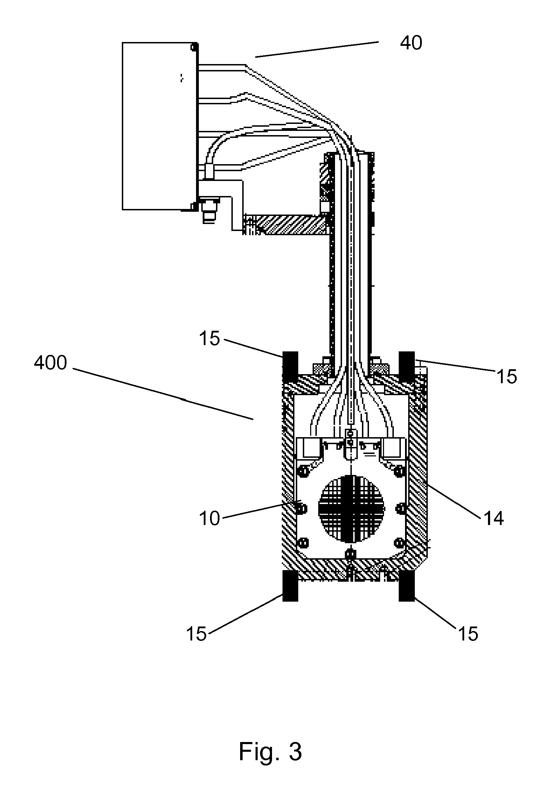

Fig. 3 shows a front view of the monitor assembly 400 and associated beam monitor electronics 40 in the direction of the arrow A indicated on Fig. 1. The monitor assembly 400 comprises a housing 14.

In an alternative embodiment, not shown in a figure, the two monitors 10, 20 forming the beam monitor assembly 400 are not mechanically linked together and the two monitors 10, 20 are supported each individually with their proper support means 30. In this embodiment the two monitors 10, 20 can each be enclosed with their proper housing 14.

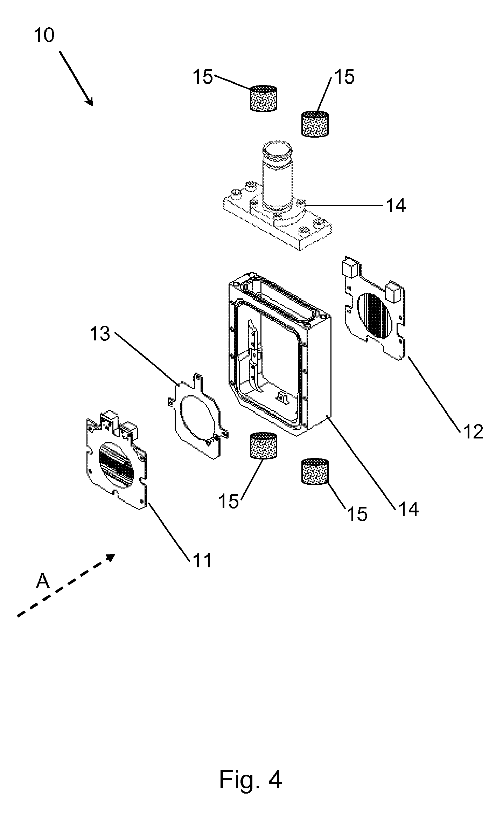

Fig. 4 shows an exploded view of the beam profile monitor 10 of the device 1 of Fig.1. The beam monitor 10 mainly comprises two anode electrodes 11, 12 consisting of conductive strips along a printed circuit board support and a cathode electrode 13 located between said anode electrodes 11, 12. Anode electrodes 11, 12 and cathode electrode 13 are both enclosed in a housing 14, the latter that provides the mechanical support and the gas volume gap. Anode electrodes 11, 12 and cathode electrode 13 form two contiguous strip ionization chambers. Strips of anode electrode 11 are arranged in a horizontal orientation while those of anode electrode 12 are arranged in a vertical orientation.

Alternatively, this order may be inversed in a device of the invention. In this manner, strips of anode electrode 11 and those of anode electrode 12 form matrix consisting of vertical and horizontal strips. When the radiation beam enters the beam monitor 10 the volume gas is ionized and resulting electrons are collected by strips of both anode electrodes 11, 12 due to the electrical field generated by anode and cathode electrodes within the beam profile monitor 10.

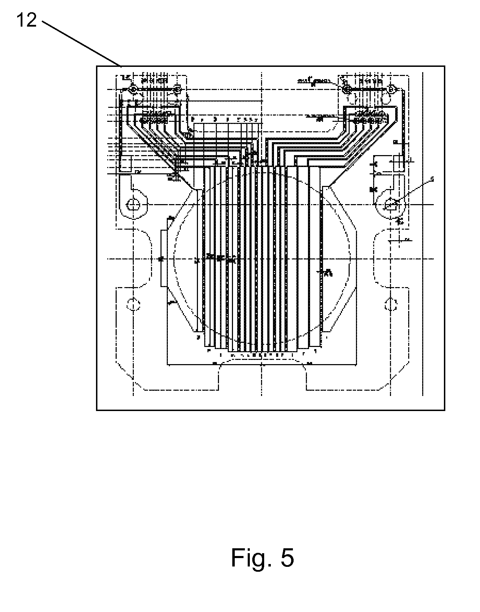

Fig. 5 shows a top view of the anode electrode 12 of the beam monitor 10 of the device 1 of Fig.1. The anode electrode 12, which forms together with the cathode electrode 13 an ionization chamber, comprises a plurality of strips where the number, the width and the orientation of the strips and the pitch between the strips may vary depending on the application.

Any suitable strip ionization chambers can be used as beam monitors in a device of the invention.

Preferably, suitable strip ionization chambers are described for example in the document "Strips ionization chambers as a 3-D detector for Particle Therapy", Brusasco et al., Nuclear Instruments and Methods in Physics Research A 389 (1997) 499-512.

Alternatively, other detectors, such as pixel ionization chambers or transmission ionization chambers (as the one described in document "DAVID- a translucent multi-wire transmission ionization chamber for in vivo verification of IMRT and conformal irradiation techniques", B. Poppe et al., Phys. Med. Biol. 51 (2006) 1237 - 1248), may also be used in a device of the invention.

Alternative embodiments can use other technologies for the beam monitors 10, 20, for example using scintillator based detectors or using scintillating optical fibers.

The sizes of the beam monitors (10, 20) must be designed to intersect the entire radiation beam delivered by the beam delivery system (500). If the beam delivery system is capable of delivering radiation beams resulting in different field sizes, the beam delivery system needs to be configured to deliver radiation beams that can be fully intersected by the two beam monitors (10,20).

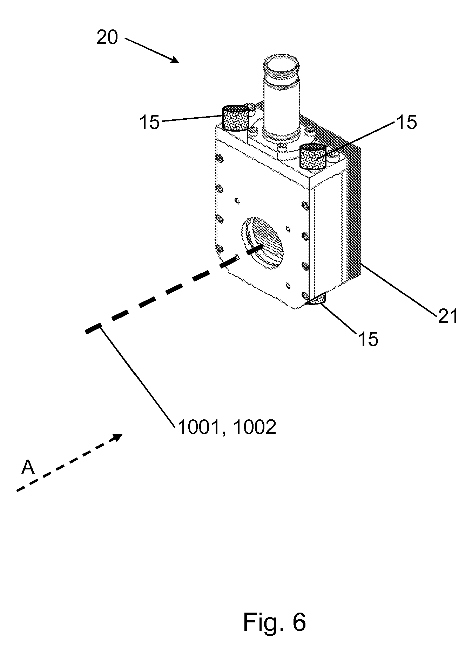

Fig.6 shows a perspective view of the other beam monitor 20 of the device 1 of Fig.1. The beam monitor 20 is identical to the beam monitor 10 but additionally comprises a beam stopping member 21 attached to the back side for preventing the particle field going beyond the monitor.

This beam stopping member 21 can have the same section as the beam profile monitor 20 with a thickness of about 10 cm and is made up of brass.

This beam stopping member 21 is removable from the beam profile monitor 20 for situations where one does not want to block the radiation beam.

In Fig. 2, the beam monitor 20 was assumed not to have a beam stopping member 21 and hence the radiation beam, represented by pointed lines, was not blocked at the level of the beam monitor assembly 400.

The two beam monitors 10,20 will intercept the radiation beam 2001, 2002 and measure a radiation field in two dimensions.

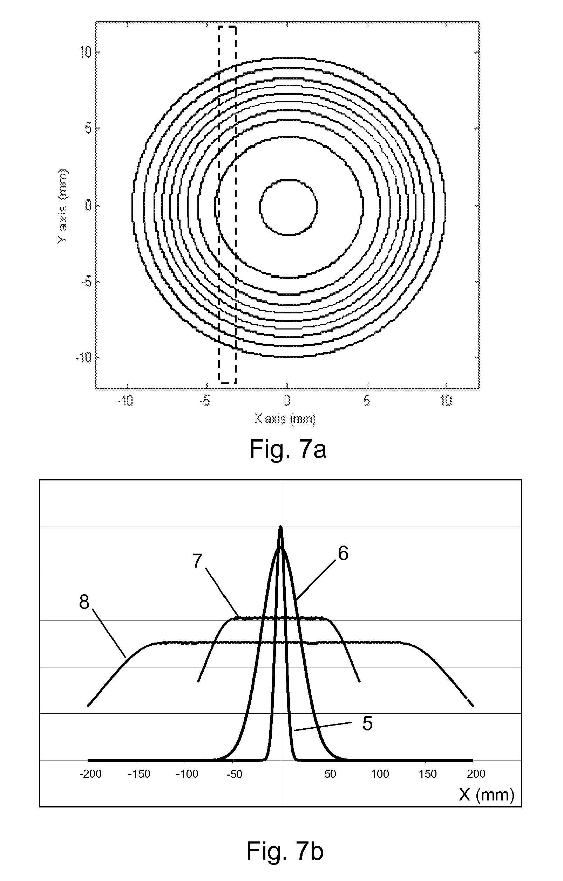

Fig 7a shows an example of the intensity or dose profile of a conical radiation beam resulting in a circular radiation field when detected with a two dimensional beam monitor 10,20. The axes X and Y are two orthogonal axes parallel with the plane of the beam monitor. The ten circles represent the contour map of the dose of the radiation field, each contour line represents a 10% step going from 100% in the center of the field down to 0 % at the outside of the field. The radiation field shown in Fig. 7a is obtained with a beam delivery system applying a passive scattering technique (double scattering) and where an aperture (i.e. a collimator) with a circular opening is installed in the beam delivery system.

Such a circular field can also be obtained with a beam delivery system applying the pencil beam scanning technique where the radiation beam is generated by scanning an elementary pencil beam across a plane perpendicular to the radiation beam axis. By properly defining the scanning trajectory of the elementary pencil beam, the resulting radiation field can have a circular or other geometrical shape.

The beam monitor 10,20 can either be a position sensitive detector (e.g. pixel chamber) measuring the response of the radiation beam in a two-dimensional plane or each beam monitor can comprise a pair of separate detectors for measuring the radiation beam in two orthogonal directions (e.g. horizontal X and vertical Y strip detectors).

When strip detectors are used, part of the two dimensional detector responses are integrated over the trip area.

The dotted line on Fig 7a represents an example of a strip area.

Hence when orthogonal strip detectors are used one will obtain radiation field profiles in two orthogonal directions.

Examples of radiation field profiles are given in Fig. 7b. The shape of the radiation field profiles will depend on the beam delivery technique used by the beam delivery system. Fig. 7b shows several examples of symmetric radiation fields that could be observed with an X strip detector: curve 6 is a radiation field resulting from a radiation beam having a Gaussian shape which is a typical beam that is delivered by a beam delivery system applying a single scattering technique, curves 7 and 8 are typical curves that are expected from a double scattering system where larger radiation fields can be obtained.

The penumbra of the profiles is influenced by the use of collimators (also called apertures) in the beam delivery system.

The penumbra is here defined as the spatial region where the radiation dose drops from 80% to 20% when compared with the dose value in the center of the radiation field.

With a pencil beam scanning beam delivery system uniform geometrical field shapes (e.g. circles, squares, rectangulars), can be created and the lateral shapes will be similar to the ones observed in double scattering. The sharpness of the edges (penumbra) will depend on the size of the elementary pencil beam.

The curve 5 on Fig. 7b is an example of an elementary radiation field resulting from an elementary pencil beam which is used in PBS. This elementary radiation field could be measured if the scanning magnets in the beam delivery system are off. However, for the preferred embodiment of the invention, when using the PBS technique, the elementary pencil beam will be scanned in such a way as to form geometrical field shapes (e.g. circle, square, ...) on the level of the beam monitor. In this way all elements in the beam delivery system, including the scanning magnets, are used in a configuration similar to what is used for patient irradiation.

When using the method and device of invention, the radiation beams to be used can either comprise mono-energetic particles or can comprise particles with various energies. This will depend on the beam delivery technique applied.

For example for a passive scattering technique where for spreading the beam along the beam direction a range modulator rotating at high speed (for example at 100Hz) is used and hence one is essentially delivering a superposition of radiation beams of various energies.

The energy of the particles is defined by the thickness of the various steps of the range modulator.

When using a PBS technique, the target volume is divided in different layers, each layer being characterised by a particle energy. The irradiation is then performed layer per layer. For the purpose of performing irradiations with PBS in the framework of the present invention, it is sufficient to irradiate only one layer and hence using a radiation beam comprising particles having essentially the same energy.

The radiation beams that are used with the method and device of the invention are symmetrical radiation beams such that when intercepting the radiation beam with a beam monitor (10,20) the resulting radiation field has a geometrical shape that easily allows to determine the center of the radiation field (e.g. circle, rectangular, ...). Radiation beams used for patient treatment are in general different from these type of geometrical radiation beams, radiation beams for patient treatment are adapted according to the shape of the patient specific target volume.

According to the preferred embodiment of this invention, processing means to analyse the shape of the radiation field profiles as detected by the beam monitors 10, 20 are provided.

For each direction, the center of the symmetric shapes is determined (e.g. through curve fitting) resulting in the coordinates of the interception points of the radiation beam axis 1001, 1002 in the planes of the beam monitor.

Based on the position of the beam monitor 10,20 in the reference 3D coordinate system, the interception point of the central trajectory 1001, 1002 with the monitor detector 10 can be determined.

The same method is used to determine the interception point of the radiation beam axis 1001, 1002 with the second particle monitor detector 20.

By knowing the two interception points the radiation beam axis in the 3D reference coordinate system 150 can be reconstructed by defining the radiation beam axis as being the line passing through the two interception points.

The width of the strips 11,12 comprised in the beam monitors 10,20 can vary in order to optimize detector efficiency, for example the width of the strips could vary from 1,5 mm in the center of the beam monitor to 3,5 mm on the edges. For determining the dimensions of the beam monitor 10, 20 the size of the radiation fields to be measured need to be taken into account.

We now discuss how the interception points of the radiation beam axis 1001, 1002 with the beam monitors 10, 20 can be determined with respect to a 3D reference coordinate system 150.

As an example we choose the FRS as the 3D reference coordinate system. The FRS which is a coordinate system fixed to the treatment room is shown on Fig. 2b.

The interception points of the radiation beam axis 1001, 1002 with the two monitors are determined as discussed above by analysing the measured profiles (see examples of profiles in Fig. 7b) in the two orthogonal directions and by determining the center of the profiles. The coordinates obtained of these center points are determined in the coordinate system associated with the beam monitor assembly (BPMCS: Beam Profile Monitor Coordinate System).

In a second step these coordinates in the BPM coordinates system are transformed are expressed in the FRS coordinate system.

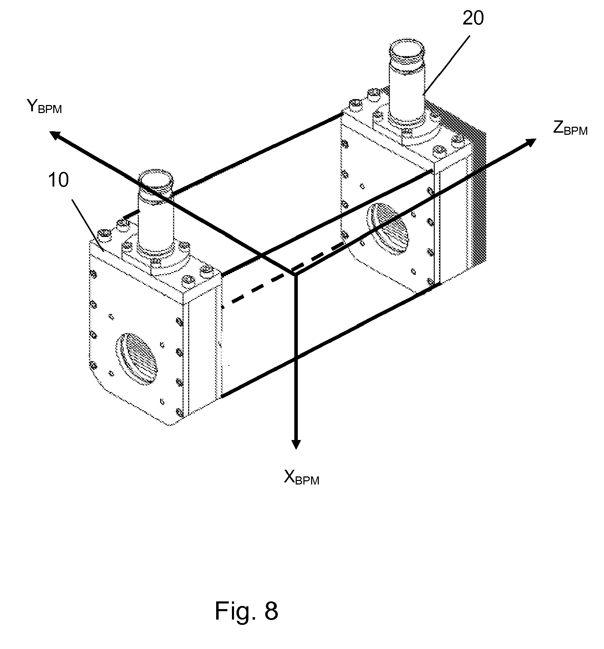

An example of a definition of the BPMCS coordinate system attached to the beam monitor assembly is shown in Fig. 8:

- The X-axis is parallel to the central strip of the anode electrodes 12 of the beam monitors 10 and 20 ;
- The Y-axis is parallel to the central strip of the second anode electrodes 11 of the beam monitors 10 and 20 ;
- The Z-axis is defined so as to have a right handed coordinate system and to have the plus direction in the direction of propagation of the proton beam;
- The origin of the this system is located in the plane halfway between the monitor detectors 10, 20.

The individual positions of each strip anode 11, 12 of the beam monitors 10,20 which are part of the beam monitor assembly are well known and defined in the BPMCS by the mechanical design of the beam detector assembly.

Hence, when defining the center position of the beam profiles of the radiation field (Fig. 7b) based on the responses of the strip detectors 10, 20, the center positions are specified in the BPMCS.

When the beam monitor assembly 400 has been setup in the treatment room the relation between the BPMCS - associated to the beam monitor assembly 400 - and the FRS - associated to the treatment room - needs to be determined.

For this purpose, the monitor assembly comprising the two beam profile monitors 10, 20 comprises a number of reference markers 15 which allow to determine the position of the beam monitor assembly 400 in the reference 3D coordinate system (e.g. FRS). The reference markers 15, shown in Fig. 1, 3, 4 and 6, can for example be theodolite targets. Therefore the coordinates of these reference markers 15 in the FRS can be precisely measured thanks to a theodolite.

The coordinates of the reference markers 15 in the BPMCS are well known from the mechanical design of the beam monitor assembly.

As a result of the theodolite measurements, a list of coordinates in the FRS for each reference marker 15 with associated coordinates in the BPMCS is obtained, this information can be stored in a configuration file of computer 50.

These data obtained from the theodolite measurements (said configuration file) are used as input data to determine the transformation matrix between BPMCS and FRS.

Such a coordinate transformation may be defined by considering a 4x4 transformation matrix M wherein the value of element $M_{i,j}$ takes into account the coordinates of the reference markers or theodolite targets 15 in the BPMCS and those in the FRS.

The matrix M is used to convert the coordinates from the BPMCS (the initial coordinate system) to the FRS (the final coordinate system).

Once the unknown components $M_{i,j}$ of the transformation matrix M have been determined, it is possible to easily convert coordinates from the BPMCS to the FRS coordinate system.

The equation below represents this coordinates transformation:

$$\begin{pmatrix} X_{FRS} \\ Y_{FRS} \\ Z_{FRS} \\ 1 \end{pmatrix} = \begin{bmatrix} M_{1,1} & M_{1,2} & M_{1,3} & M_{1,4} \\ M_{2,1} & M_{2,2} & M_{2,3} & M_{2,4} \\ M_{3,1} & M_{3,2} & M_{3,3} & M_{3,4} \\ 0 & 0 & 0 & 1 \end{bmatrix} \cdot \begin{pmatrix} X_{BPM} \\ Y_{BPM} \\ Z_{BPM} \\ 1 \end{pmatrix} \qquad \text{Equation 1}$$

When the 12 components of the transformation matrix M are known, it will be possible to convert the coordinates of the interception points of the central beam line 1001, 1002 with the monitors 10, 20 from the BPMCS to the FRS coordinate system.

In practice, the twelve matrix components are not all linearly independent, indeed the transformation can be described by three translations (Tr(X,Y,Z)) and three rotations ($R_X$, $R_Y$, $R_Z$) and hence the number of unknowns is reduced to six:

$$M(X, Y, Z, \theta_Z, \theta_Y, \theta_X) = R_Y(\theta_Y) . R_Z(\theta_Z) . . R_X(\theta_X) . Tr(X, Y, Z) \qquad \text{Equation 2}$$

The transformation matrix components can be computed using an iterative algorithm in order to minimize a goodness of fit function (GOF). The advantage of minimizing a GOF instead of solving an algebraic equation is that it is possible to take into account the experimental error in the measurement of the theodolite target position with the theodolite.

The solution minimizes the deviation to the measurement and therefore includes the measurement error in the computations.

In addition, it is possible to use a large number of measurements that would make the system over-determined in a algebraic solution.

The theoretical model is the position of the ith target in the FRS CS computed from the target position in the BPM CS:

$$\begin{pmatrix} X_{FRS}^{T_i} \\ Y_{FRS}^{T_i} \\ Z_{FRS}^{T_i} \\ 1 \end{pmatrix} = M(X, Y, Z, \theta_Z, \theta_Y, \theta_X) . \begin{pmatrix} X_{BPM}^{T_i} \\ Y_{BPM}^{T_i} \\ Z_{BPM}^{T_i} \\ 1 \end{pmatrix} \qquad \text{Equation 3}$$

The transformation matrix M is computed using the Equation 2.

The Goodness of fit function is the sum of the square of the differences between the target position measured with the theodolite (($X^{Ti}_{mes}$, $Y^{Ti}_{mes}$, $Z^{Ti}_{mes}$) and the model position given by Equation 3:

$$GOF(X, Y, Z, \theta_Z, \theta_Y, \theta_X) = \sum_{i=1}^{N} \left( \left( X_{mes}^{T_i} - X_{FRS}^{T_i} \right)^2 + \left( Y_{mes}^{T_i} - Y_{FRS}^{T_i} \right)^2 + \left( Z_{mes}^{T_i} - Z_{FRS}^{T_i} \right)^2 \right)$$

$$\text{Equation 4}$$

The sum is carried out over all the available measured positions of the reference markers 15.

The parameters (X, Y, Z, $\theta_Z$, $\theta_Y$, $\theta_X$) must be optimized (for example using a Maquard Levenberg algorithm) until the GOF function reaches a minimum.

The iterative optimization algorithm selects a set of value for the parameters (X, Y, Z, $\theta_Z$, $\theta_Y$, $\theta_X$). It uses Equation 3 to compute the theoretical target position in the FRS from the target position expressed in the BPMCS (information available in the said configuration file). Then it uses Equation 4 to compute the value of the GOF function.

A minimum number of target positions on the beam monitor assembly is required in order to have sufficient data to determine the orientation and position of the BPMCS coordinate system in the FRS.

In order to determine the orientation of one coordinate system with respect to another coordinate system it is necessary to have at least 3 non-collinear reference markers 15.

Using the transformation matrix M the coordinates in the FRS of the interception points of the central beam line 1001, 1002 with the two beam monitors can be determined and hence the central beam line can be reconstructed in the FRS. For a gantry system having multiple beam delivery directions the same process described above needs to be applied to determine for each direction the central beam line in the FRS.

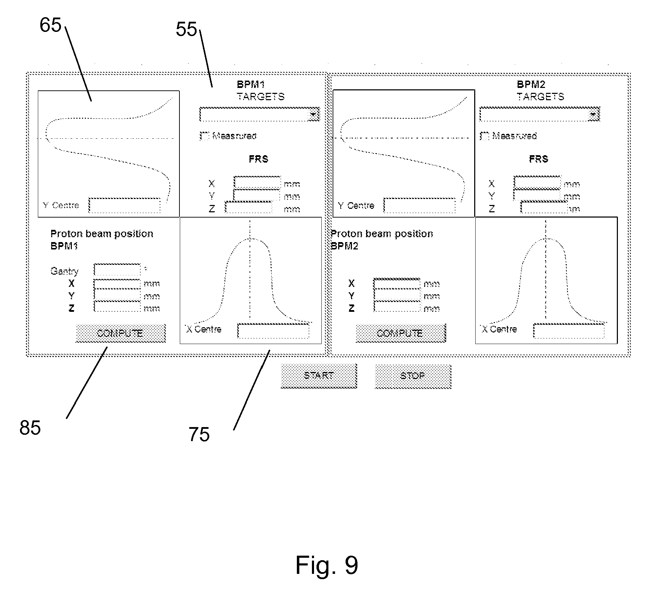

Fig. 9 shows an example of a graphical user interface of computer 50 that can be used to support the calculation of the interception points of the radiation beam axis with the beam monitors 10, 20.

This interface is divided in two identical areas, each dedicated to one of the two beam monitors 10, 20.

Each area is divided in four zones and two buttons:

- the first zone of interface 55 contains edit boxes X,Y,Z to enter the with the theodolite measured coordinates of the reference markers 15 in the FRS. By selecting with the drop down box the various measured positions of the reference markers 15, multiple coordinates in the FRS of multiple reference markers 15 can be entered.
- the second zone of interface 65 represents the measured profile of the radiation field along the Y-axis of the BPMCS for one of the beam monitors 10, 20. A histogram plot of the number of counts on each strip of the beam monitor is plotted on the graph;
- the third zone of interface 75 represents the measured profile of the radiation field along the X-axis of the BPMCS for one of the beam monitors 10, 20. A histogram plot of the number of counts on each strip of the beam profile monitor is plotted on the graph;
- the fourth zone of interface 85 contains a compute button. When hitting the compute button, the computation of the transformation from the BPMCS to the FRS is performed and as a result the X,Y,Z coordinates of the interception of the radiation beam axis with the beam monitor in the FRS coordinate system is displayed.

- START / STOP buttons are used to start and stop the display of the beam profile in real time in the X and Y beam profile zones.

As discussed above, in the preferred embodiment the two monitors 10, 20 are mechanically coupled by a single frame or housing 14 which allows to define a single BPMCS coordinate system associated to the beam monitor assembly 400 comprising the two beam monitors 10,20.

In an alternative embodiment where the two beam monitors are not mechanically coupled and have both their proper housing 14 and proper setup means or support frame 30, a BPMCS needs to be defined for each beam monitor 10, 20 separately.

A set of reference markers 15 is then associated to each of the two beam monitors 10,20 separately and the position of these markers in the FRS needs to be determined with a theodolite.

In this case where each beam monitor has his proper BPMCS, the coordinates of the interception points of the central beam trajectory line 1001, 1002 with the two beam monitors 10, 20 needs to be determined in the BPMCS coordinate system of each respective beam monitor 10, 20.

In a second step, transformations of the coordinates needs to be made from the BPMCS of each beam monitor 10, 20 to the FRS.

The same method can be applied as described above to determine the respective transformation matrices.

It is now further discussed how to determine the coordinates of the radiation isocenter 101, 102 in the FRS.

According to a preferred embodiment we discuss first the case where the beam delivery system 500 is mounted on an isocentric gantry.

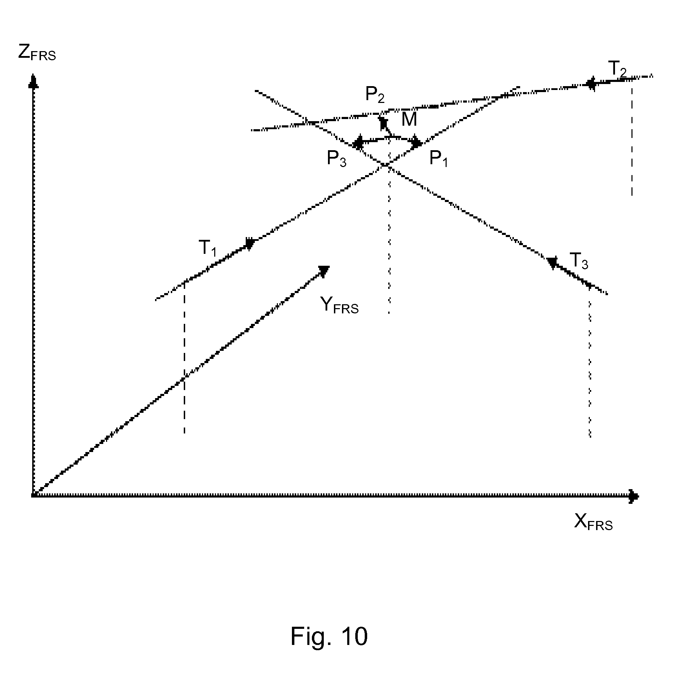

In Fig. 10 a schematical representation in the FRS coordinate system of three radiation beam axes $(T_1, T_2, T_3)$ corresponding to three different delivery directions $(D_i)$ of the beam delivery system, i.e. for three different gantry angles, is given.

The final step is now to determine for each beam delivery direction, the FRS coordinates of the radiation isocenter 101,102.

Several methods exist to determine the radiation isocenter.

The points $P_i$ on Fig. 10 are determined as follows: In a first step a point M in the FRS is determined which is a "common radiation isocenter" for each gantry angle and in a second step each point $P_i$ is defined as the orthogonal projection of the point M on the corresponding radiation beam axis $T_i$.

These points $P_i$ are the radiation isocenters 101, 102 specified in the FRS for each of the orientations of the beam delivery system (i.e. gantry angle for the case of a gantry system).

To determine the common radiation isocenter (M) in the first step, several methods are valid.

Point M can for example be determined as being the point with the smallest average distance to all radiation beam axes.

A second example to determine point M is to determine a sphere that intersects or is at least tangent to all radiation beam axes, the center point of this sphere is then point M, the common radiation isocenter.

Alternatively, instead of determining for each direction ($D_i$ , $D_j$) the coordinates in the FRS of the radiation isocenters 101, 102 one can also determine the coordinates in the FRS of point M and then define the relative positions between point M and points $P_i$. Thanks to this method a correction vector with respect to the common radiation isocenter (M) for each delivery direction $D_i$ is obtained.

The determination of a radiation isocenter position for each gantry angle or the determination of the relative deviations of the radiation isocenter from a common radiation isocenter (M) as function of gantry angle can further be used to correct the patient couch as function of gantry angle.

The device of invention can also be used for a fixed beam line system where one or more fixed beam lines are available in the treatment room.

In this a case the same process is followed to determine the radiation beam axis in the FRS as described before. The radiation isocenter of a fixed beam line can for example be defined along the radiation beam axis at a well defined distance from the beam delivery system.

A reference point or plane needs to be identified on the beam delivery system in order to determine this distance.

A reference can for example be the mechanical frame of the nozzle which can have specific reference marks. The position of this frame in the reference coordinate system can then be determined by measuring the position of the reference marks with a theodolite.

As the radiation beam axis is known in the reference coordinate system one can then identify the coordinates of a point on the radiation beam axis which is positioned at a given distance from the reference frame. This point is then by definition the radiation isocenter. If there is more than one fixed beam line in the treatment room the same method can be repeated to determine the radiation isocenter for each beam line in the treatment room.

The device and method of the invention can also be used for an excentric gantry system, a system where there is no common radiation isocenter and where the radiation isocenter needs to be determined for each gantry angle.

As a conclusion, many advantages are obtained with the method and device of the invention:

- The position of the radiation isocenter can be determined for each available beam delivery direction.
- The radiation isocenter is determined using the beam delivery system in a configuration that is equivalent to a treatment configuration, i.e. potential effects on the radiation isocenter due to potential misalignment of multiple elements in the beam delivery system (e.g. scanning magnets, collimators, scatter elements, ...) need to be taken into account.
- The device and method of the invention allows to measure the radiation isocenter for each particle radiation technique (e.g. single scattering, double scattering, pencil beam scanning, wobbling).
- The measurements of the radiation isocenter can be performed fast and accurate.
- The device and method of the invention can be used with a particle therapy system where multiple types of particles (e.g. protons and carbon ions) can be delivered in the same treatment room. In such type of treatment rooms the radiation isocenter for protons and carbon ions can vary and the device and method of the invention allows the measurement of these different radiation isocenters.

**Numbering**

| | |
|---|---|
| 1 | DEVICE |
| 10,20 | BEAM MONITORS |
| 11, 12 | ANODE ELECTRODE |
| 13 | CATHODE ELECTRODE |
| 14 | HOUSING |
| 15 | REFERENCE MARKER |
| 21 | BEAM STOPPING MEMBER |
| 30 | SUPPORT FRAME |
| 40 | BEAM MONITOR ELECTRONICS |
| 50 | COMPUTER |
| 55 | FIRST ZONE OF INTERFACE |
| 65 | SECOND ZONE OF INTERFACE |
| 75 | THIRD ZONE OF INTERFACE |
| 85 | FOURTH ZONE OF INTERFACE |
| 101, 102 | RADIATION ISOCENTER |
| 150 | 3D REFERENCE COORDINATE SYSTEM |

| 400 | BEAM MONITOR ASSEMBLY |
|---|---|
| 500 | BEAM DELIVERY SYSTEM |
| 1001, 1002 | RADIATION BEAM AXIS |
| 2001, 2002 | RADIATION BEAM |

**Claims**

1. A method for determining the position of a radiation isocenter (101, 102) of a particle beam delivery system (500) with respect to a 3D reference coordinate system (150), said particle beam delivery system (500) being configured for delivering a radiation beam (2001, 2002) along one or a multiple number of delivery directions ($D_1$, $D_2$, ...., $D_i$, ..., $D_n$) in a treatment room, the method comprising the steps of:

   a) positioning the particle beam delivery system (500) in one of the delivery directions ($D_1$, $D_2$, ...., $D_i$, ..., $D_n$);
   b) setting up at least two beam monitors (10, 20) downstream of the said particle beam delivery system (500),
   c) establishing the position and orientation of the said two beam monitors (10, 20) with respect to the 3D reference coordinate system;
   d) delivering said radiation beam (2001, 2002);
   e) measuring detector responses of said two beam monitors (10,20);
   f) reconstructing the radiation beam axis (1001, 1002) of said radiation beam (2001, 2002) with respect to said 3D reference coordinate system (150);
   g) determining for each of said delivery directions ($D_1$, $D_2$, ...., $D_i$, ..., $D_n$) the position of the corresponding said radiation isocenter with respect to said 3D reference coordinate system (150).

2. A method according to claim 1, **characterized in that** said step of determining for each of said delivery directions ($D_1$, $D_2$, ...., $D_i$, ..., $D_n$) the position of the corresponding said radiation isocenter with respect to said 3D reference coordinate system (150) further comprises steps of:

   a) defining the coordinates of the common radiation isocenter (M) for all said beam delivery directions;
   b) computing the coordinates of the radiation isocenter (101,102) for each said delivery directions by making an orthogonal projection of said common radiation isocenter (M) on the corresponding radiation beam axis (1001, 1002).

3. A method according to claim 1, **characterized in that** said step of determining for each of said delivery directions ($D_1$, $D_2$, ...., $D_i$, ..., $D_n$) the position of the corresponding said radiation isocenter with respect to said 3D reference coordinate system (150) further comprises steps of:

   a) defining the coordinates of the common radiation isocenter (M) for all said beam delivery directions;
   b) determining the position of the said radiation isocenter (101, 102) by computing for each said beam delivery directions a correction vector with respect to said common radiation isocenter (M).

4. A method according to claim 1, **characterized in that** said step of determining for each of said delivery directions ($D_i$, $D_2$, ...., $D_i$, ..., $D_n$) the position of the corresponding said radiation isocenter with respect to said 3D reference coordinate system (150) further comprises the step of:

   determining the position of said radiation isocenter (101,102) for each said delivery direction as being the point on said radiation beam axis (1001, 1002) located at a given distance from said beam delivery system (500).

5. A device (1) for determining the position of a radiation isocenter (101, 102) of a particle beam delivery system (500) with respect to a 3D reference coordinate system (150) , said particle beam delivery system (500) being configured for delivering a radiation beam (2001, 2002) along one or a multiple number of delivery directions ($D_i$, $D_2$, ...., $D_i$, ..., $D_n$) in a treatment room, the device (1) being **characterized in that** it comprises:

   o a beam monitor assembly (400) comprising at least two beam monitors (10, 20) arranged in a face to face relationship at a given distance from each other, each of said two beam monitors being generating two-dimensional detector responses when said radiation beam (2001, 2002) is crossing the said two beam monitors (10,20);
   o beam monitor electronics (40) for performing data acquisition of said detector responses;
   o means to set up the said beam monitor assembly (400) downstream of the said particle beam delivery system (500) in such a manner that said radiation beam (2001, 2002) is crossing the two said beam monitors when said particle beam delivery system (500) is delivering said radiation beam along said delivery direction;

o processing means (50) to reconstruct the radiation beam axis (1001, 1002) of said radiation beam (2001, 2002) with respect to said 3D reference coordinate system (150);

o processing means (50) for computing and determining for each of said delivery directions ($D_1$, $D_2$, ...., $D_i$, ..., $D_n$) the position of the corresponding said radiation isocenter (101,102) with respect to said 3D reference coordinate system (150), said computing using the said reconstructed radiation beam axis (1001, 1002) of each said delivery direction.

6. The device according to claim 5 wherein said processing means to reconstruct the radiation beam axis (1001, 1002) further comprises:

o processing means to analyse the said detector responses and to identify the intersection points of the radiation beam axis (1001, 1002) with said two beam monitors (10, 20);

o processing means to compute and determine the position of the said intersection points with respect to the said 3D reference coordinate system.

7. The device (1) according to claims 5 or 6 wherein each of said beam monitors (10, 20) comprises a two-dimensional detector.

8. The device (1) according to claim 7 wherein said two-dimensional detector comprises a first strip electrode (11) and a second strip electrode (12) arranged in a face to face relationship and having respective strips oriented in two orthogonal directions whereby forming a matrix of orthogonal strips.

9. The device (1) according to any of claims 5 to 8 wherein said beam detector assembly (400) comprises reference markers (15) for computing and determining the position of said two beam monitors (10, 20) with respect to a said reference coordinate system.

10. The device (1) according to any of the claims 5 to 9 wherein said setup means comprises rotation means for adjusting the orientation of said beam detector assembly with respect to the said delivery direction of said radiation beam (2001, 2002).

11. The device (1) according to any of claims 5 to 10 wherein said setup means comprises means to attach said detector assembly directly to a patient couch (700).

Fig. 1

Fig. 2a

Fig. 2b

Fig. 3

Fig. 4

Fig. 5

Fig. 6

Fig. 7a

Fig. 7b

$Y_{BPM}$

20

10

$Z_{BPM}$

$X_{BPM}$

Fig. 8

Fig. 9

Fig. 10

Europäisches
Patentamt

European
Patent Office

Office européen
des brevets

# EUROPEAN SEARCH REPORT

Application Number

EP 08 16 9193

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | US 2006/002519 A1 (JENKINS TODD P [US] ET AL JENKINS TODD PERRIN [US] ET AL) 5 January 2006 (2006-01-05) * abstract * * figures 1B,6,7,9,10 * * claim 17 * | 1,5-9,11 | INV. A61N5/10 |
| A | | 2-4,10 | |
| A | WO 2004/031803 A (SUNNYBROOK AND WOMEN S COLLEGE [CA]) 15 April 2004 (2004-04-15) * claim 1 * | 8 | |
| A | MUTIC SASA ET AL: "Quality assurance for computed-tomography simulators and the computed-tomography-simulation process: Report of the AAPM Radiation Therapy Committee Task Group No. 66" MEDICAL PHYSICS, AIP, MELVILLE, NY, US, vol. 30, no. 10, 1 October 2003 (2003-10-01), pages 2762-2792, XP012011963 ISSN: 0094-2405 * figure 6 * | 10 | |

| | TECHNICAL FIELDS SEARCHED (IPC) |
|---|---|
| | A61N G01T |

The present search report has been drawn up for all claims

| Place of search | Date of completion of the search | Examiner |
|---|---|---|
| The Hague | 6 February 2009 | Schembri, Valentina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 08 16 9193

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2009

| Patent document cited in search report | Publication date | Patent family member(s) | | Publication date |
|---|---|---|---|---|
| US 2006002519 A1 | 05-01-2006 | WO | 2006007584 A2 | 19-01-2006 |
| WO 2004031803 A | 15-04-2004 | AU | 2003273659 A1 | 23-04-2004 |
| | | CA | 2501058 A1 | 15-04-2004 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7349523 B

**Non-patent literature cited in the description**

- **Brusasco et al.** Strips ionization chambers as a 3-D detector for Particle Therapy. *Nuclear Instruments and Methods in Physics Research A,* 1997, vol. 389, 499-512

- **B. Poppe et al.** DAVID- a translucent multi-wire transmission ionization chamber for in vivo verification of IMRT and conformal irradiation techniques. *Phys. Med. Biol.,* 2006, vol. 51, 1237-1248